# FASCICULE DE BREVET EUROPEEN

(11) **EP 1 090 626 B1**
(45) Date de publication et mention de la délivrance du brevet: **19.06.2002**
(21) Numéro de dépôt: 00402319.8
(22) Date de dépôt: 18.08.2000
(51) Int. Cl.: A61K 7/48, A61K 7/06, A61K 7/02, A61K 7/00, A61K 7/50

(54) **Composition sous forme d'émulsion huile-dans-eau contenant des fibres, et ses utilisations notamment cosmétiques**
O/W-Emulsion enthaltende Fibern und ihre Verwendung in der Kosmetik
O/W-Emulsion containing fibres and its use in cosmetics

(30) Priorité: 07.10.1999 FR 9912505
(43) Date de publication de la demande: 11.04.2001
(73) Titulaire: L'OREAL, 75008 Paris (FR)
(72) Inventeur: Afriat, Isabelle, 75003 Paris (FR)
(74) Mandataire: Rasson, Catherine

(56) Documents cités:
- US-A- 5 498 407
- US-A- 5 728 389
- PATENT ABSTRACTS OF JAPAN vol. 012, no. 110 (C-486), 8 avril 1988 (1988-04-08) & JP 62 238211 A (KOBAYASHI KOOC:KK), 19 octobre 1987 (1987-10-19)
- PATENT ABSTRACTS OF JAPAN vol. 006, no. 266 (C-142), 25 décembre 1982 (1982-12-25) & JP 57 158714 A (SHISEIDO KK), 30 septembre 1982 (1982-09-30)

## Description

La présente demande se rapporte à une composition sous forme d'émulsion huile-dans-eau contenant des fibres et un système tensioactif particulier, et à l'utilisation de la dite composition, en particulier pour le soin, le traitement et/ou le maquillage de la peau du corps ou du visage, des cheveux, des cils et/ou des lèvres.

Il est connu par le document JP07-196440 des compositions cosmétiques contenant des fibres de polyamide courtes, celles-ci donnant aux dites compositions un toucher velouté et une bonne tenue cosmétique. Toutefois, l'incorporation de ces fibres de polyamide dans les émulsions huile dans eau (H/E) pose des problèmes de stabilité, c'est-à-dire que les émulsions déphasent à température ambiante ou à des températures plus élevées, et ce notamment quand la quantité de fibres est importante.

Il subsiste donc le besoin d'émulsions H/E contenant des fibres, et notamment des fibres de polyamide, et présentant de bonnes propriétés cosmétiques sans avoir les inconvénients de l'art antérieur.

La demanderesse a découvert de façon inattendue que le système tensioactif comprenant au moins un ester de glycéryle et d'acide gras en C₈-C₂₄ et au moins un ester de polyéthylène glycol et d'acide gras en C₈-C₂₄, permettait de réaliser des émulsions huile-dans-eau contenant des fibres, stables.

Ainsi, la présente invention concerne une composition sous forme d'émulsion huile-dans-eau comprenant, dans un milieu physiologiquement acceptable, une phase huileuse dispersée dans une phase aqueuse, caractérisée en ce qu'elle contient des fibres et un système tensioactif comprenant au moins un ester de glycéryle et d'acide gras en C₈-C₂₄ et au moins un ester de polyéthylène glycol et d'acide gras en C₈-C₂₄.

On entend ici par « milieu physiologiquement acceptable », un milieu compatible avec la peau, les lèvres, le cuir chevelu, les cils, les yeux et/ou les cheveux.

La composition obtenue selon l'invention se présente sous forme d'une crème (produit souple par opposition à un produit solide). Elle présente une bonne stabilité dans le temps, même à une température supérieure à la température ambiante (par exemple 45°C) et elle a une texture veloutée, agréable à l'application.

Les fibres utilisables dans la composition de l'invention peuvent être des fibres hydrophiles ou hydrophobes, d'origine synthétique ou naturelle, minérale ou organique. Elles peuvent être courtes ou longues, unitaires ou organisées par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 10 nm à 5 mm et mieux de 0,1 mm à 1,5 mm. Leur section peut être comprise dans un cercle de diamètre allant de 2 nm à 100 µm, de préférence allant de 20 nm à 20 µm ou de 5 µm à 50 µm. Le poids des fibres est souvent donné en denier ou décitex.

Les fibres peuvent être celles utilisées dans la fabrication des textiles et notamment des fibres de soie, de coton, de laine, de lin, de cellulose extraites notamment du bois, des légumes ou des algues, de polyamide (Nylon®), de rayonne, de viscose, d'acétate notamment d'acétate de rayonne, de poly-p-phénylène téréphtamide notamment de Kevlar®, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Les fibres utilisables dans la composition selon l'invention sont de préférence des fibres de polyamide et/ou de poly-p-phénylène-téréphtamide. Leur longueur peut aller de 0,1 à 5 mm, de préférence de 0,25 à 1,6 mm, et leur diamètre moyen peut aller de 5 à 50 µm. En particulier, on peut utiliser les fibres de polyamide commercialisées par les Etablissements P. Bonte sous le nom Polyamide 0,9 Dtex 0,3 mm, ayant un diamètre moyen de 6 µm, un poids d'environ 0,9 dtex et une longueur allant de 0,3 mm à 1,5 mm. On peut aussi utiliser les fibres de poly-p-phénylène-téréphtamide de diamètre moyen de 12 µm et de longueur d'environ 1,5 mm comme celles vendues sous le nom de Kevlar Floc par la société Du Pont Fibres. Selon un mode particulier de réalisation de l'invention, ces fibres sont introduites dans la phase huileuse de l'émulsion.

Les fibres peuvent être présentes dans la composition selon l'invention en une quantité allant de 0,1 à 20 % en poids et de préférence de 0,5 à 10 % en poids par rapport au poids total de la composition.

Le système tensioactif de l'émulsion selon l'invention comprend au moins un ester de glycéryle et d'acide gras en C₈-C₂₄ et au moins un ester de polyéthylène glycol et d'acide gras en C₈-C₂₄.

L'ester de glycéryle et d'acide gras peut être obtenu notamment à partir d'un acide comportant une chaîne alkyle linéaire saturée, ayant de 8 à 24 et de préférence de 16 à 22 atomes de carbone. Comme ester de glycéryle et d'acide gras, on peut citer notamment le stéarate de glycéryle (mono-, di- et/ou tri-stéarate de glycéryle), le ricinoléate de glycéryle et leurs mélanges.

L'ester de polyéthylène glycol et d'acide gras peut être obtenu notamment à partir d'un acide comportant une chaîne alkyle linéaire saturée, ayant de 8 à 24 et de préférence de 16 à 22 atomes de carbone. Il peut comporter un ou plusieurs groupes oxyéthylénés (chaînes de polyéthylène glycol : OE), et par exemple de 1 à 150 groupes oxyéthylénés. Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate).

L'ester de glycéryle et d'acide gras peut être présent en une quantité allant de préférence de 0,5 à 5 % en poids et mieux de 2 à 3 % en poids par rapport au poids total de la composition.

L'ester de polyéthylène glycol et d'acide gras peut être présent en une quantité allant de préférence de 0,5 à 5 % en poids et mieux de 2 à 3 % en poids par rapport au poids total de la composition.

Le système tensioactif est globalement présent dans la composition selon l'invention en une quantité allant de 1 à 10 % en poids, de préférence 4 à 6 % en poids par rapport au poids total de la composition. Ce système tensioactif est généralement incorporé dans la phase huileuse.

La phase huileuse de la composition selon l'invention représente généralement de 10 à 50 % et de préférence de 15 à 30 % en poids par rapport au poids total de la composition.

La phase huileuse peut être constituée par tous les corps gras et notamment les huiles, classiquement utilisés dans les domaines cosmétique ou dermatologique.

Parmi les huiles utilisables dans l'émulsion de l'invention, on peut citer par exemple les huiles végétales telles que les huiles de jojoba, avocat, amande douce, abricot, maïs et la fraction liquide de beurre de karité ; les huiles minérales comme l'huile de vaseline, le polyisobutène hydrogéné (huile de parléam); les huiles de synthèse comme le palmitate d'éthyl-2 hexyle, le myristate d'isopropyle, l'isoparaffine hydrogénée, l'isononanoate d'isononyle, l'octanoate de cétéaryle ; les huiles de silicone volatiles ou non volatiles et les huiles fluorées. Les autres corps gras susceptibles d'être présents dans la phase huileuse peuvent être par exemple les acides gras et les alcools gras tels que l'alcool cétylique.

La phase aqueuse de la composition de l'invention constitue en général de 50 à 90 % et de préférence de 60 à 80 % en poids par rapport au poids total de la composition.

De façon connue, les compositions de l'invention peuvent contenir des adjuvants habituels dans les domaines considérés, tels que des actifs hydrophiles ou lipophiles, des conservateurs, des gélifiants, des antioxydants, des parfums, des solvants, des charges ou des nacres, des filtres, des matières colorantes (pigments ou colorants solubles), des agents basiques ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme actifs, on peut citer par exemple les hydratants tels que les polyols comme la glycérine et le sorbitol ; les agents kératolytiques ; les dépigmentants ; les amincissants, et tout actif approprié pour le but final de la composition.

Selon la fluidité de la composition que l'on souhaite obtenir, on peut y ajouter un ou plusieurs gélifiants hydrophiles ou lipophiles. Comme gélifiants hydrophiles, on peut citer par exemple les polymères carboxyvinyliques, tels que les carbomers. Comme gélifiants lipophiles, on peut citer les argiles modifiées telles que les bentones telles que le mélange « cyclomethicone, Quaternium-18 hectorite, SD alcohol 40 » (10/85/5) (nom CTFA) commercialisé sous la dénomination Bentone Gel VS-5 par la société Rheox ; les organopolysiloxanes élastomères réticulés tels que ceux commercialisés sous les noms KSG6 de Shin-Etsu, Trefil E-505C ou Trefil E-506C de Dow-Coming, Gransil de Grant Industries (SR-CYC, SR DMF10, SR-DC556), ou ceux commercialisés sous forme de gels: KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric.

Ces gélifiants lorsqu'ils sont présents, sont généralement utilisés à des concentrations allant de 0,1 à 7 % et de préférence de 0,1 à 5 % en poids de matière active par rapport au poids total de la composition.

Les compositions, objets de l'invention, trouvent leur application dans un grand nombre de traitements notamment cosmétiques et peuvent ainsi constituer une composition cosmétique, notamment pour le traitement, la protection, le soin, le démaquillage et/ou le nettoyage de la peau, des lèvres et/ou des cheveux, et/ou pour le maquillage de la peau des lèvres, des cils et/ou du corps.

Les compositions selon l'invention peuvent par exemple être utilisées comme produits de soin, de démaquillage et/ou de nettoyage pour le visage sous forme de crèmes ou de laits ou comme produits de maquillage (peau, cils et lèvres) par incorporation de pigments ou de colorants, par exemple comme fonds de teint.

Aussi, l'invention a pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau, des lèvres, des cils et/ou du corps.

L'invention a aussi pour objet un procédé de traitement cosmétique de la peau, y compris du cuir chevelu, des cheveux, des cils et/ou des lèvres, caractérisé par le fait qu'on applique sur la peau, les cheveux, les cils et/ou les lèvres, une composition telle que définie ci-dessus.

Les exemples qui suivent permettront de mieux comprendre l'invention, sans toutefois présenter un caractère limitatif. Les quantités indiquées sont en % en poids, sauf mention contraire.

### Exemple 1 : Crème protectrice de jour

| *Phase huileuse* | |
|---|---|
| Huile de vaseline | 6,2 % |
| Myristate d'isopropyle | 3 % |
| Alcool cétylique | 7 % |
| Stéarate de glycéryle | 2,5 % |
| PEG-50 stearate | 2,5 % |
| | |

| *Phase aqueuse :* | |
|---|---|
| Conservateurs | 0,3 % |
| Eau déminéralisée | qsp 100 % |
| | |
| Fibres de polyamide (Polyamide 0,9 Dtex, 0,3 mm - Société Paul Bonte) | 10 % |

Mode opératoire : On fait chauffer la phase huileuse jusqu'à ce qu'elle soit homogène, puis on y ajoute les fibres. On verse ensuite le mélange obtenu dans la phase aqueuse préalablement chauffée à la même température, sous forte agitation.

On obtient une crème qui reste stable dans le temps, même après conservation à 45°C. A l'application sur la peau, elle est d'une grande douceur et est particulièrement adaptée pour assouplir la peau.

### Exemple 2 : Crème protectrice de jour

| *Phase huileuse* | |
|---|---|
| Huile de parléam | 6,2 % |
| Myristate d'isopropyle | 3 % |
| Alcool cétylique | 7 % |
| Stéarate de glycéryle | 2,5 % |
| PEG-50 stearate | 2,5 % |
| | |

| *Phase aqueuse :* | |
|---|---|
| Conservateurs | 0,3 % |
| Eau déminéralisée | qsp 100 % |
| | |
| Fibres de polyamide (Polyamide 0,9 Dtex, 0,3 mm - Société Paul Bonte) | 10 % |

Le mode opératoire est le même que celui de l'exemple 1.

On obtient une crème apte à hydrater la peau.

### Exemple 3 : Crème protectrice de jour

| *Phase huileuse* | |
|---|---|
| Huile d'abricot | 6,2 % |
| Myristate d'isopropyle | 3 % |
| Alcool cétylique | 7 % |
| Stéarate de glycéryle | 2,5 % |
| PEG-50 stearate | 2,5 % |
| | |

| *Phase aqueuse :* | |
|---|---|
| Conservateurs | 0,3 % |
| Eau déminéralisée | qsp 100 % |
| | |
| Fibres de polyamide (Polyamide 0,9 Dtex, 0,3 mm - Société Paul Bonte) | 10 % |

Le mode opératoire est le même que celui de l'exemple 1.

On obtient une crème conférant à la peau de la douceur.

## Revendications

1. Composition sous forme d'émulsion huile-dans-eau comprenant dans un milieu physiologiquement acceptable, une phase huileuse dispersée dans une phase aqueuse, **caractérisée en ce qu'**elle contient des fibres et un système tensioactif comprenant au moins un ester de glycéryle et d'acide gras en C₈-C₂₄ et au moins un ester de polyéthylène glycol et d'acide gras en C₈-C₂₄.

2. Composition selon la revendication 1, **caractérisée en ce que** les fibres ont une longueur allant de 0,1 à 5 mm.

3. Composition selon la revendication 1 ou 2, **caractérisée en ce que** les fibres ont une section comprise dans un cercle de diamètre allant de 5 µm à 50 µm.

4. Composition selon l'une quelconque des revendications précédentes, **caractérisée en ce que** les fibres sont choisies parmi les fibres de polyamide, les fibres de poly-p-phénylène-téréphtamide et leurs mélanges.

5. Composition selon l'une quelconque des revendications précédentes, **caractérisée en ce que** les fibres sont présentes en une quantité allant de 0,1 % à 20 % en poids par rapport au poids total de la composition.

6. Composition selon l'une quelconque des revendications précédentes, **caractérisée en ce que** l'ester de glycéryle et d'acide gras est choisi dans le groupe comprenant les mono-, di- et/ou tri-stéarates de glycéryle, le ricinoléate de glycéryle et leurs mélanges.

7. Composition selon l'une quelconque des revendications précédentes, **caractérisée en ce que** l'ester de polyéthylène glycol et d'acide gras comporte de 1 à 150 groupes oxyéthylénés.

8. Composition selon l'une quelconque des revendications précédentes, **caractérisée en ce que** le système tensioactif est présent en une quantité allant de 1 à 10 % en poids par rapport au poids total de la composition.

9. Composition selon l'une quelconque des revendications précédentes, **caractérisée en ce que** la phase huileuse représente de 10 à 50 % en poids par rapport au poids total de la composition.

10. Composition selon l'une quelconque des revendications précédentes, **caractérisée en ce qu'**elle constitue une composition cosmétique.

11. Utilisation cosmétique de la composition selon l'une quelconque des revendications 1 à 10, pour le traitement, la protection, le soin, le démaquillage et/ou le nettoyage de la peau, des lèvres et/ou des cheveux, et/ou pour le maquillage de la peau, des lèvres, des cils et/ou du corps.

12. Procédé de traitement cosmétique de la peau, des cheveux, des cils et/ou des lèvres, **caractérisé par le fait qu'**on applique sur la peau, les cheveux, les cils et/ou les lèvres, une composition selon l'une quelconque des revendications 1 à 10.

## Patentansprüche

1. Zusammensetzung, die in Form einer Öl-in-Wasser-Emulsion vorliegt und die in einem physiologisch akzeptablen Medium eine in einer wäßrigen Phase dispergierte Ölphase enthält, **dadurch gekennzeichnet, daß** sie Fasern und ein Tensidsystem aufweist, das mindestens einen Glycerylester einer C₈₋₂₄-Fettsäure und mindestens einen Polyethylenglykolester einer C₈₋₂₄₋Fettsäure enthält.

2. Zusammensetzung nach Anspruch 1, **dadurch gekennzeichnet, daß** die Fasern eine Länge von 0,1 bis 5 mm aufweisen.

3. Zusammensetzung nach Anspruch 1 oder 2, **dadurch gekennzeichnet, daß** die Fasern einen Querschnitt aufweisen, der in einen Kreis mit einem Durchmesser von 5 bis 50 µm einbeschrieben werden kann.

4. Zusammensetzung nach einem der vorhergehenden Ansprüche, **dadurch gekennzeichnet, daß** die Fasern unter den Polyamidfasern oder den Fasern aus Poly-p-phenylenterephthamid oder deren Gemischen ausgewählt sind.

5. Zusammensetzung nach einem der vorhergehenden Ansprüche, **dadurch gekennzeichnet, daß** die Fasern in einem Mengenanteil von 0,1 bis 20 Gew.-%, bezogen auf das Gesamtgewicht der Zusammensetzung, vorliegen.

6. Zusammensetzung nach einem der vorhergehenden Ansprüche, **dadurch gekennzeichnet, daß** der Fettsäureglycerylester unter Glycerylmonostearat, Glyceryldistearat und/oder Glyceryltristearat, Glycerylricinoleat und deren Gemischen ausgewählt ist.

7. Zusammensetzung nach einem der vorhergehenden Ansprüche, **dadurch gekennzeichnet, daß** der Fettsäurepolyethylenglykolester 1 bis 150 Ethylenoxidgruppen enthält.

8. Zusammensetzung nach einem der vorhergehenden Ansprüche, **dadurch gekennzeichnet, daß** der Tensidsystem in einer Menge von 1 bis 10 Gew.-%, bezogen auf das Gesamtgewicht der Zusammensetzung, vorliegt.

9. Zusammensetzung nach einem der vorhergehenden Ansprüche, **dadurch gekennzeichnet, daß** die Ölphase 10 bis 50 Gew.-%, bezogen auf das Gesamtgewicht der Zusammensetzung, ausmacht.

10. Zusammensetzung nach einem der vorhergehenden Ansprüche, **dadurch gekennzeichnet, daß** es sich um eine kosmetische Zusammensetzung handelt.

11. Kosmetische Verwendung der Zusammensetzung nach einem der Ansprüche 1 bis 10 zur Behandlung, zum Schutz, zur Pflege, zum Abschminken und/oder zur Reinigung der Haut, der Lippen und/oder der Haare und/oder zum Schminken der Haut, der Lippen, der Wimpern und/oder des Körpers.

12. Verfahren zur kosmetischen Behandlung der Haut, der Haare, der Wimpern und/oder der Lippen, **dadurch gekennzeichnet, daß** auf die Haut, die Haare, die Wimpern und/oder die Lippen eine Zusammensetzung nach einem der Ansprüche 1 bis 10 aufgetragen wird.

## Claims

1. Composition in oil-in-water emulsion form comprising, in a physiologically acceptable medium, an oily phase dispersed in an aqueous phase, **characterized in that** it contains fibres and a surfactant system comprising at least one glyceryl ester of a C₈-C₂₄ fatty acid and at least one polyethylene glycol ester of a C₈-C₂₄ fatty acid.

2. Composition according to Claim 1, **characterized in that** the fibres have a length ranging from 0.1 mm to 5 mm.

3. Composition according to Claim 1 or 2, **characterized in that** the fibres have a cross section within a circle of diameter ranging from 5 µm to 50 µm.

4. Composition according to any one of the preceding claims, **characterized in that** the fibres are chosen from polyamide fibres, poly-p-phenylene terephthamide fibres and mixtures thereof.

5. Composition according to any one of the preceding claims, **characterized in that** the fibres are present in an amount ranging from 0.1 to 20% by weight relative to the total weight of the composition.

6. Composition according to any one of the preceding claims, **characterized in that** the glyceryl ester of a fatty acid is chosen from the group comprising glyceryl mono-, di- and/or tristearate and glyceryl ricinoleate, and mixtures thereof.

7. Composition according to any one of the preceding claims, **characterized in that** the polyethylene glycol ester of a fatty acid comprises from 1 to 150 oxyethylenated groups.

8. Composition according to any one of the preceding claims, **characterized in that** the surfactant system is present in an amount ranging from 1 to 10% by weight relative to the total weight of the composition.

9. Composition according to any one of the preceding claims, **characterized in that** the oily phase represents from 10 to 50% by weight relative to the total weight of the composition.

10. Composition according to any one of the preceding claims, **characterized in that** it constitutes a cosmetic composition.

11. Cosmetic use of the composition according to any one of Claims 1 to 10, for treating, protecting, caring for, removing make-up from and/or cleansing the skin, the lips and/or the hair, and/or for making up the skin, the lips, the eyelashes and/or the body.

12. Cosmetic treatment process for the skin, the hair, the eyelashes and/or the lips, **characterized in that** a composition according to any one of Claims 1 to 10 is applied to the skin, the hair, the eyelashes and/or the lips.
